# EUROPEAN PATENT APPLICATION

(11) **EP 4 455 669 A1**
(43) Date of publication of application: **30.10.2024**
(21) Application number: 21968850.4
(22) Date of filing: 21.12.2021
(51) Int. Cl.: G01N 33/576

(54) **METHOD FOR MEASURING GLYCOSYLATED SURFACE ANTIGEN PROTEIN OF HBV**

(71) Applicant: RCMG Inc., Tsukuba-shi, Ibaraki 305-0047 (JP)
(72) Inventor: NARIMATSU Hisashi, Tsukuba-shi, Ibaraki 305-0047 (JP); ANGATA Kiyohiko, Tsukuba-shi, Ibaraki 305-0047 (JP); YONEYAMA Hiroyuki, Tsukuba-shi, Ibaraki 305-0047 (JP)
(74) Representative: Grünecker Patent- und Rechtsanwälte PartG mbB
(86) International application number: PCT/JP2021/047294
(87) International publication number: WO 2023/119413

(57) **Abstract**

The present invention relates to a method which is a method for detecting hepatitis B virus comprising a step of bringing a sample into contact with an antibody which binds to a surface antigen protein, and a lectin.

## Description

### TECHNICAL FIELD

The present invention relates to a kit for measuring hepatitis B virus (HBV) antigen protein on the glycosylated surface specifically and simply.

### BACKGROUND ART

Hepatitis B virus (HBV) which infects about 2 billion people worldwide and about 300 million people chronically, causes chronic hepatitis, cirrhosis, and liver cancer, accounting for 20 to 30% of all liver cancer cases and 880,000 deaths annually (Non-Patent Documents 1 to 5). Although vaccines against HBV exist, about 30 million people are still newly infected annually, and 4.5 million people continue to receive treatment for chronic hepatitis (Non-Patent Documents 1 and 2). While the goal of HBV treatment is undoubtedly to improve life expectancy and quality of life, the specific clinical goal of treatment is the disappearance of quantitative hepatitis B surface antigen protein (qHBsAg: quantitative hepatitis B surface antigen) in the blood (Non-Patent Documents 6 to 8). However, the current treatment with interferon preparations and nucleic acid analogue preparations rarely leads to the disappearance of hepatitis B virus surface antigen protein (HBsAg: hepatitis B surface antigen), and the development of new treatments has been very active again (Non-Patent Documents 6 to 8).

The envelope protein of HBsAg is constituted by an S protein (S-HBs protein, S=small), an M protein (M-HBs protein, M=middle) and an L protein (L-HBs protein, L=large). Existing diagnostic reagents for HBsAg detection recognize the S protein (S-HBs) possessed by all HBV particles, and have therefore become indispensable test drugs in daily clinical practice as a broad indicator to confirm the presence of HBV infection.

It has been known for a long time that HBVs, like other viruses, are bound to glycans, but recent MS (mass spectrometry) analysis has revealed their glycan structures (Non-Patent Documents 9 and 10). In S-HBs, one N-glycosylated chain is added to asparagine (N146) at the 146^{th} amino acid, but not all N146 is attached to the glycosylated chain, and the ratio of the glycosylated chain attachment is not clear. In addition, it is also known that the N-glycosylated chain are modified by sialic acid. It has been experimentally demonstrated that the N-glycosylated chain attached to S-HBs plays a role in secretion of HBV particles outside the cell (including into the blood) (Non-Patent Documents 11 to 13).

On the other hand, the biggest problem of N-glycosylation in clinical practice is that it is related to evasion of immunity of the virus host, such as humans (Non-Patent Document 14). Since HBV is released into the blood after invading host cells and wearing host-derived glycosylated chain, and thus it is considered a mechanism to escape from elimination by the immune system, as a "self' glycosylated chain from the point of view of the host immune cells. In addition, it is also considered a mechanism that the spreading branches (branch of glycosylated chain) of the N-glycosylated chain may cover the antigen recognition sites of the virus, thus preventing it from being recognized by the immune system. Further, viruses themselves change the added position of glycosylated chain by mutation. For example, it is well known that glycine (G130) at the 130^{th} amino acid is replaced by asparagine (G130N), resulting in ectopic addition of glycosylated chains.

Existing HBV vaccines (Bimmugen, Heptavax) are produced using "non-glycosylated chain-modification" S-HBs as the immunogen. In the existing vaccine innoculated patient serum, antibodies against S-HBs are produced, but the epitopes recognized by the antibodies bind to the non-glycosylated S-HBs portion. It has also been shown that the glycosylated portions are not recognized (Non-Patent Document 15). Although it has been experimentally shown that antibodies against such S-HBs can neutralize normal HBV, it has also been shown that they are unable to neutralize glycosylated chain mutant strains (Non-Patent Document 16). Thus, the fact that the therapeutic effect of S-HBs varies greatly depending on the glycosylation of S-HBs is a major clinical problem.

In the existing reagents for detecting HBsAg, used is "non-glycosylated chain-modification" S-HBs. Accordingly, there may be considered cases in which viruses with mutated glycosylated chain may not be detected by the existing tests and may test negative (which actually exists as "occult infection"), and the case causing immune evasion by glycosylation mechanism may not be accurately identified so that the treatment may be inadequate. At present, no reagents for detecting glycosylated S-HBs have been established.

Analysis itself of the glycosylated chain attached to S-HBs is possible by MS, and in fact their structures have been clarified by MS. However, it is still too time-consuming and costly to process a large number of patient sera in clinical practice, and is not yet practical. Further, since patient sera contain a large number of glycosylated chain derived from dietary sources and those derived from various organ metabolisms glycosylated chain exist, even if it were possible to comprehensively grasp the glycosylated chain alone, it would be technically difficult to identify HBV-derived glycosylated chain.

### PRIOR ART DOCUMENTS

### NON-PATENT DOCUMENTS

1) WHO. Guidelines for the prevention, care and treatment of persons with chronic hepatitis B infection. March 2015.
2) WHO. Guidelines on hepatitis B and C testing. February 2017.
3) Terrault NA, Loch ASF, McMahon BJ et al. Update on prevention, diagnosis, and treatment of chronic hepatitis B: AASLD 2018 hepatitis B guidance. Hepatol 67: 1560, 2018.
4) European association foe the study of the liver. EASL 2017 clinical practice guideline on the management of hepatitis B virus infection. J Hepatol 67: 370, 2017.
5) Japan Society of Hepatology, Committee for the Preparation of Guidelines for Hepatitis Treatment. Hepatitis B Treatment Guidelines (Version 3.1). March 2019
6) Lok AS, Zoulim F, Dusheiko G et al. Hepatitis B cure: From discovery to regulatory approval. J Hepatol 66: 1296, 2017.
7) Revill PA, CHisari FV, Block jM et al. A global scientific strategy to cure hepatitis B. Lancet Gastroenterol Hepatol. 4: 545, 2019.
8) Cornberg M, Lok AS. Terrault NA et al. Guidance for design and endpoints of clinical trials in chronic hepatitis B - Report from the 2019 EASL-AASLD HBV treatment endpoints conference. J Hepatol 72: 539, 2020.
9) Schmitt S et al. Structure of pre-S2 N- and O-linked glycans in surface proteins from different genotypes of hepatitis B virus. Journal of General Virology (2004), 85, 2045-2053.
10) Wagatsuma T, Kuno A, Angata K et al. Highly sensitive glycan profiling of hepatitis B viral particle and a simple methods for Dane particle enrichment. Anal Chem 90: 10196, 2018.
11) Lu X et al. Aberrant trafficking of hepatitis B virus glycoproteins in cells in which N-glycan processing is inhibited. Proc. Natl. Acad. Sci. USA (1997) 94, 2380-2385.
12) Mehta A et al. α-Glucosidase inhibitors as potential broad based anti-viral agents. FEBS Letters (1998) 430, 17-22.
13) Ito K et al. Impairment of hepatitis B virus virion secretion by single-amino-acid substitutions in the small envelope protein and rescue by a novel glycosylation site. Journal of Virology (2010) 12850-12861
14) Dobrica MO, Lazar C, Branza-Nichita N. N-Glycosylation and N-Glycan Processing in HBV Biology and Pathogenesis. Cells. 2020 Jun 4;9(6):1404. doi: 10.3390/cells9061404. PMID: 32512942; PMCID: PMC7349502.
15) Tajiri K et al. Analysis of the epitope and neutralizing capacity of human monoclonal antibodies induced by hepatitis B vaccine. Antiviral Research (2010) 87, 40-49.
16) Hamada-Tsutsumi S et al. Validation of cross-genotype neutralization by hepatitis B virus-specific monoclonal antibodies by in vitro and in vivo infection. PLoS ONE (2015) 10: e0118062.

### SUMMARY OF THE INVENTION

### PROBLEMS TO BE SOLVED BY THE INVENTION

An object of the present invention is to provide a method for measuring hepatitis B virus (HBV) specifically, easily, and with high accuracy.

### MEANS TO SOLVE THE PROBLEMS

For detection of glycoproteins such as a glycosylated S-HBs, antibodies that detect the glycopeptide as epitope (anti-glycopeptide antibody) can be considered, but it is still difficult to detect HBV having "self-glycosylated chain", and there has been no successful attempt to develop an antibody for detection. In addition, because the glycosylated chain-added portion changes due to mutation, even if antibodies against a specific glycopeptide sequence are developed, it is not always possible to detect the mutated glycosylated S-HBs with only a single antibody. On the other hand, since lectins are often used for the detection of the glycosylated chain, we have examined whether the combination of lectins against N-glycosylated chain and S-HBs antibodies can detect glycosylated S-HBs or not.

The inventors found that SSA lectin (Salvia sclarea agglutinin) recognizes α-2,6-sialic acid on S-HBs by using lectin-blotting technique (Non-Patent Documents 10). Thus, an ELISA system by combination of a lectin and an antibody was developed and attempted to detect SSA lectin-positive S-HBs using actual sera from chronic hepatitis B patient, but the specificity of the reaction was unexpectedly low and the system was not suitable for detection. It can be considered the possibility that it may react with background components such as glycosylated chains present in normal sera, etc., and it was suggested that it would be difficult to detect this with SSA lectin in actual human sera.

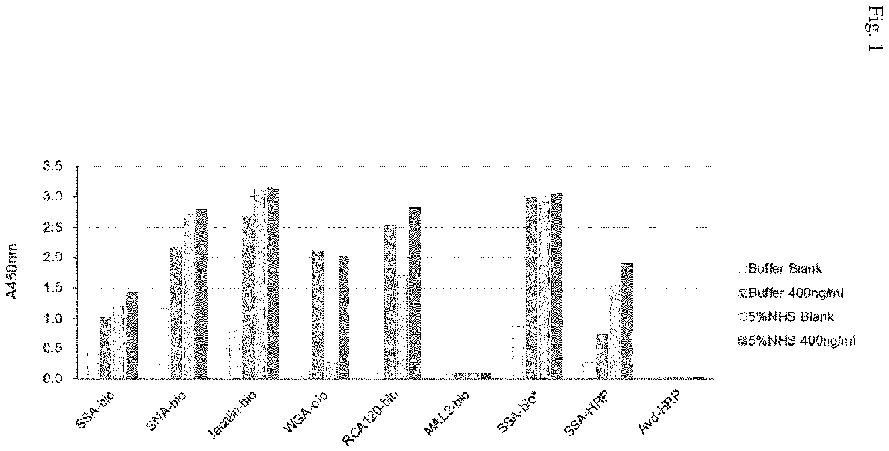

Thus, the inventors began by independently designing lectin-antibody combinations using actual serum from chronic hepatitis B patients and conducted extensive research, and could be clarified surprisingly that the combination of wheat germ agglutinin (WGA lectin (wheat germ agglutinin)) and an antibody could specifically detect the antibody (Fig. 1). This finding means that the glycosylated chains attached to S-HBs bind to WGA, which is completely unexpected.

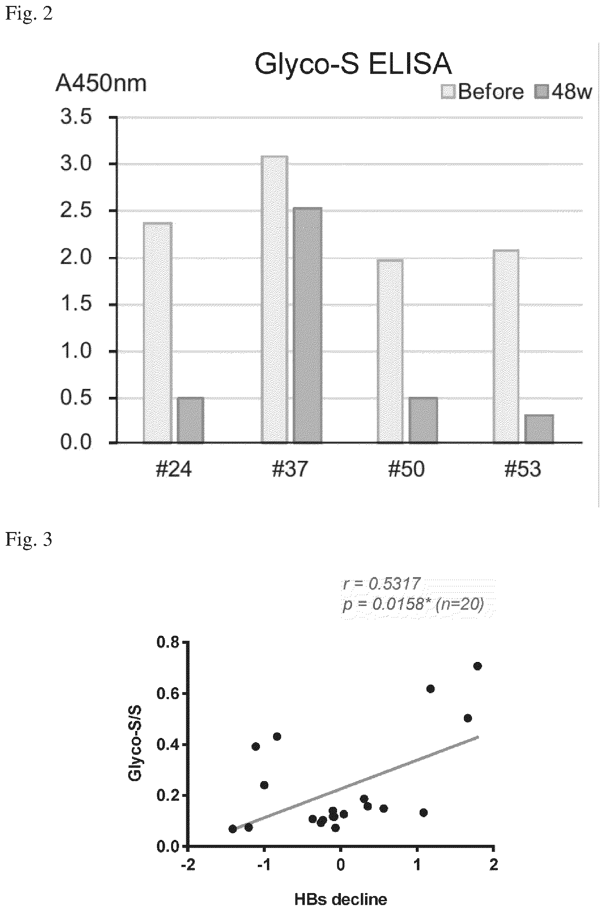

Further, when serum from a different patient group was measured before and after treatment using the WGA-S-HBs combination, it was possible to measure both, and the changes before and after the treatment were also measurable, so that it was suggested that it is useful for understanding the effect of the treatment (Fig. 2).

Using sera from the same patient population as mentioned above, the ratio of the amount of glycosylated S-HBs in the total S-HBs was determined as the ratio of "glycosylated S-HBs/S-HBs". Hereinafter, this ratio is described as the Glyco-S/S ratio. The Glyco-S/S ratio before the treatment could be easily determined (Fig. 3), and it was revealed that the glycosylated chain ratio could be measured as a continuous quantitative numerical value, even when compared with conventional MS, etc. That is, the value of the glycosylated surface antigen protein, which is the numerator, is determined by the detection using a lectin and an antibody, and the value of the surface antigen protein, which is the denominator, is determined by antibody detection without using a lectin.

The goal of treatment is the "disappearance" of qHBsAg, but it is difficult to achieve the disappearance by the current treatment alone, and long-term observation over several decades is required. Therefore, as an indicator to determine the efficacy of treatment, a "decrease" in sHBsAg is used. Above all, a decrease of log 10 of 1 or more is considered to be a clinically significant decrease, and is used worldwide as an endpoint of drug efficacy in the development of new drugs (Non-Patent Documents 7).

Using serum from the same patient population as mentioned above, when the correlation between the Glyco-S/S ratio before the treatment and the HBsAg lowering degree after the treatment was examined, then the surprising result was obtained that the higher the Glyco-S/S ratio before the treatment, the more glycosylation on the S-HBs protein, and the lower the HBsAg lowering degree, that is, the more resistant the patient was to treatment (Fig. 3). When the Glyco-S/S value is at least 0.5 or more, that is, more than half of the glycans are attached, the therapeutic effect is deemed to be reduced.

It has previously been known that glycosylation of S-HBs is important for immune evasion, but it was not known at all whether what degree of glycosylation is applied, and whether the degree of glycosylation affects the efficacy of immunotherapy or resistance. The present invention makes it possible to quantitatively measure the content and ratio of the glycosylation of S-HBs by immunological methods, instead of the conventional methods such as MS, etc. By quantifying detection of glycosylation simply and quickly, whereas detection of glycosylation was previously qualitative and time-consuming and costly, this will contribute to the evaluation and prediction of the relationship between the degree of glycosylation and therapeutic effects, particularly in therapeutic effects of immunotherapy, which have been little known until now. In addition, it can detect infections that have been overlooked due to changes in mutations and glycosylation, and thus has an extremely high contribution to clinical practice.

More specifically, the present invention is to provide the following [1] to [11].
[1] A method which is a method for detecting hepatitis B virus (HBV) comprising a step of bringing a sample into contact with an antibody which binds to a surface antigen protein and a lectin.
[2] The detecting method described in [1], wherein the surface antigen protein of hepatitis B virus is S protein.
[3] The detecting method described in [1] or [2], wherein the lectin is wheat germ agglutinin.
[4] The detecting method described in any of [1] to [3], which includes a step of measurement by an ELISA method.
[5] The detecting method described in any of [1] to [4], which is for testing, prophylaxis or planning treatment of hepatitis B virus.
[6] The detecting method described in any of [1] to [5], which is for screening medicines for testing, prophylaxis or treatment of hepatitis B virus.
[7] The detecting method described in any of [1] to [6], which further includes a step of calculating a glycosylated surface antigen protein/surface antigen protein.
[8] The detecting method described in any of [1] to [7], wherein the sample is derived from a patient who is receiving or has received nucleic acid analog therapy.
[9] The detecting method described in any of [1] to [8], wherein the sample is derived from a patient whose HBV-DNA suppression in blood has been confirmed.
[10] A kit for detecting a glycosylated surface antigen protein of hepatitis B virus which comprises an antibody that binds to the surface antigen protein of hepatitis B virus, and a lectin.
[11] A method for treating or preventing hepatitis B, including the method described in any of [1] to [9].

### EFFECTS OF THE INVENTION

In patients with chronic hepatitis B (CHB), glycosylation of S-HBs, which all HBV particles possess, is involved in immune evasion from the host and is a major barrier to treatment aiming at eliminating HBsAg. And yet, the fact that the site of glycosylation also changes with mutation makes the therapy more difficult. Although conventional MS can identify the glycosylation structure, it is time-consuming and costly, and is not suitable for the analysis of a large amount of actual clinical serum samples. In addition, although it is possible to comprehensively view a huge amount of glycans in blood, it is contrary difficult to selectively determine the glycans related to diseases.

According to the immunological methods of the present invention, that is, antigen-antibody reaction and glycosylated chain-lectin reaction, it could be firstly clarified that S-HBs coated by N-glycosylated chain among S-HBs can be detected rapidly and simply, and quantitatively, and further, in actual patient serum, the degree of glycosylation on S-HBs (content or ratio of glycosylated S-HBs among S-HBs) is significantly related to the therapeutic response.

The glycosylated chain that modifies S-HBs of the CHB patient is the glycosylated chain derived from individual patients. According to the present invention, the degree of glycosylation of S-HBs can be easily and quantitatively determined, which is useful for determining therapeutic strategies and, especially for patient stratification of new drugs for immunological mechanisms and diagnosis of therapeutic effects, that is, personalized medicine. In addition, it can find patients having mutations that would be overlooked by the conventional detecting agents that detect HBsAg "having no glycosylated chain", so that it can be also expected to contribute to reducing potential horizontal infections.

### BRIEF DESCRIPTION OF THE DRAWINGS

Fig. 1 shows the results of combination ELISA of various kinds of lectin and antibodies against HBs antigens. The Y-axis indicates the absorbance of the ELISA. SSA could not detect N-glycosylated S-HBs as a specific positive signal, but a very clear detection was achieved by the combination using WGA.
Fig. 2 shows the results of ELSIA of the amount of N-glycosylated S-HBs (Glyco-S) in the serum of CHB patients (n=4) before and after treatment with nucleic acid analog preparations measured by the WGA-S-HBs combination ELSIA. The Y-axis indicates the absorbance of the ELISA. Before: before treatment, 48W: 48 weeks after treatment.
Fig. 3 shows the correlation plot between the Glyco-S/S ratio before the treatment and the degree of reduction of HBsAg lowering degree after the treatment in CHB patient (n=20) before and after nucleic acid analogue treatment. The Y-axis indicates the ratio, and the X-axis indicates the degree of reduction in qHBsAg (log U/mL) after 48 weeks of treatment. A reduction of -1 or more is considered to be therapeutically effective.

### EMBODIMENTS TO CARRY OUT THE INVENTION

Hereinafter, the present invention will be explained in more detail.

The present inventor has developed a method for specifically and simply measuring glycosylated surface antigen proteins of hepatitis B virus (HBV), and more particularly, a detection and quantification method using an immunological technique that uses a combination of an antibody that binds to S-HBs protein and a lectin that binds to the glycan attached to the S-HBs protein, and has found that glycosylated S-HBs can be quantitatively measured.

In the present invention, "hepatitis B" is also expressed as HBV, and may be any of chronic hepatitis B, acute hepatitis B and fulminant hepatitis B, and "hepatitis B virus" means a virus having an ability of causing these types of hepatitis B.

In the present invention, the detection target may be any "hepatitis B virus surface antigen protein", particularly among hepatitis B virus surface antigen proteins, it may be an S protein, M protein or L protein, and preferably an S protein. The "glycosylated S-HBs" refers to a glycosylated S protein among HBV surface antigen protein that contain a glycosylation chain.

The antibody in the present invention may be any antibody that recognizes the hepatitis B virus surface antigen, and may be either a polyclonal antibody or a monoclonal antibody. In addition, the antibody of the present invention may be any antibody that can be prepared by a conventional method in the field of the art, or may be a commercially available antibody.

The lectin in the present invention may be any lectin as long as it is N-linked glycosylation chain-binding lectin such as a plant lectin, a C-type lectin, galectin and Siglec, etc., and preferably wheat germ agglutinin. In addition, the lectin of the present invention may be any lectin that can be prepared by a conventional method in the field of the art, or may be a commercially available lectin.

The antibody and lectin in the present invention can also be prepared by general methods known in the field of the art.

The present invention is to provide a detecting method of hepatitis B virus, particularly hepatitis B virus glycosylated surface antigen protein, including a step of bringing a sample into contact with an antibody which binds to a surface antigen protein, and a lectin.

The contact of the antibody which binds to a surface antigen protein, and the lectin with the sample may be carried out each simultaneously, stepwisely or successively, preferably operated by the ELISA method and the binding is measured.

The preparation method and detection method of the above-mentioned glycosylated S-HBs measurement reagent of the present invention may be the conventional method in the field of the art. The preparation of the S-HBs antibody or the WGA lectin, and the ELISA method and chemiluminescence method using them can be appropriately carried out by a person skilled in the art using general immunological testing methods in the relevant technical field. Also, it is easily possible for a person skilled in the art to adapt the present invention to various types of measuring instruments. Further, it is easily possible for a person skilled in the art to prepare a simple and rapid test kit using the finger stick method, etc.

In the present invention, the subjects of the test are all individuals infected with HBV. This applies to all patients who are being treated with HBV therapeutic agents as a matter of course, as well as to those who have stopped treatment and are being monitored, and to asymptomatic carriers.

By understanding the HBV infectious capacity, it can be used in all aspects of HBV treatment, such as judgement of the effectiveness of treatment, selection of the therapeutic agents, decision of the course of treatment, judgement whether to continue or discontinue treatment, etc.

In the present invention, as the subject of the test, it can be used for healthy individuals who are at high risk of HBV infection. Specifically, health care workers, family members of HBV-infected persons, etc. (all populations listed in WHO guidelines on hepatitis B and C testing 2017).

In the present invention, as the subject of the test, it can be used for blood donors. It contributes to prevention of horizontal infection, etc.

In the present invention, as the subject of the test, it can be used for pregnant women. It contributes to prevention of vertical zone infection, etc.

In the present invention, as the subject of the test, it can be used for preoperative checkups before surgical operations and preoperative checkups before endoscopic examinations, etc. It contributes to determine whether or not a risk of horizontal infection arises from the examination or surgery to be performed.

In the present invention, as the subject of the test, it can be used for patients who reveal liver disorder such as fatty liver, etc. By differentiating patients in whom HBV infection overlaps or is hidden behind liver disorder, it contributes to modification of the treatment strategy and improvement of patient quality of life.

The subject of the test in the present invention is all adults. In the prior art techniques, it has been reported that those who can be diagnosed to be HBV infection are only 10% of true HBV infected person (Literature: Polaris Observatory Collaborators Global prevalence, treatment, and prevention of hepatitis B virus infection in 2016: a modeling study. Lancet Gastroenterol Hepatol 3: 383-403, 2018), so that it would also be very meaningful to carry out diagnosis as an option to all adults.

The samples derived from the above-mentioned subject, patient or infected person in the present invention may be any biological samples, and there may be mentioned body fluids or tissue extracts such as blood, serum, saliva, semen, vaginal secretion and wound exudate from a subject suspected of being infected with hepatitis B virus, and in consideration of simplicity of sample acquisition and handling, blood, serum and saliva are preferred.

According to the detection method of the present invention, glycosylated H-HBs in samples derived from patients with chronic hepatitis B virus, HBV patients before starting therapeutic agents including interferon preparations or nucleic acid analog preparations, after starting, or 1 week, 2 weeks, 4 weeks, 8 weeks, 16 weeks, 48 weeks, or 48 weeks or more after starting, patients with confirmed suppression of HBV-DNA in blood, patients who have disappeared HBs antigen, patients who do not disappear HBs antigen during their lifetime, and patients before and after the administration of a new drug in the development stage, can be advantageously detected. The samples may be any biological samples. It is particularly advantageous if the samples are derived from patients who are or have been treated with nucleic acid analog preparations.

The present invention relates to a method for providing data for planning prophylaxis or treatment of HBV, comprising the step of bringing the above-mentioned antibody and lectin into contact with a sample. According to the present invention, it is possible to provide data that can serve as indicators for planning prevention or treatment of HBV, such as recurrence of symptoms and presence or absence of infectivity, which was difficult to do with the conventional techniques.

The present invention further relates to a method for screening medicines for prophylaxis or treatment of HBV, which comprises a step of bringing a sample into contact with the above-mentioned antibody and a lectin. According to the present invention, it is possible to screen for candidate drugs for the prevention or treatment of HBV based on a more accurate indicator that can reflect the degree of N-glycosylated chain added to S-HBs.

Incidentally, all prior art references cited in the present specification are incorporated in the present specification by reference.

### EXAMPLES

Hereinafter, the present invention will be more concretely explained by using Examples. However, the technical range of the present invention is not limited by these Examples.

### <Example 1>

A purified antibody against HBs antigen protein (HBsAgGi) was immobilized on a plate, and a sandwich ELISA system was constructed to examine the optimal lectin to be used for detecting the capturedHBs antigen.
1) HBsAgGi (5 µg/mL) diluted with BSA (5 µg/mL)/PBS was added to a Nunc Immobilizer Amino Plate at a volume of 0.1 mL per well, and the plate was shaken at room temperature for 4 hours to immobilize the antibody. After washing twice with PBS-T, 0.3 mL of TBS was added per well and the plate was allowed to stand overnight at 4°C for blocking.
2) After washing with PBS-T, glycosylated recombinant HBs antigen protein was diluted to 400 ng/mL with Sample dilution buffer (3% BSA/TBS-T), 0.1 mL thereof was added per well, and the plate was shaken at room temperature for 2.5 hours to be captured the antigen on the antibody solid-phased plate. In order to confirm the effect of the serum, recombinant HBs antigen to which 5% Normal human serum (NHS) was added was also treated in the same manner.
3) After washing the wells four times with PBS-T, the wells were reacted with the following lectins diluted with PBS-T at room temperature for 2 hours by shaking.
   Jacalin-biotin (1 µg/mL), MAL II-biotin (1 µg/mL), RCA120-biotin (1 µg/mL), SNA-biotin (1 µg/mL), SSA-biotin (1 µg/mL), WGA-biotin (1 µg/mL)
4) After washing four times with PBS-T, the wells were reacted with Peroxidase-conjugated Streptavidin (1/20,000 dilution) or SSA-HRP (2 µg/mL) diluted with PBS-T at room temperature for 1 hour by shaking.
5) After washing the wells four times with PBS-T, 0.1 mL of 1Step Ultra TMB-ELISA was added per well and allowed to react for 5 min, then 50 µL of 1 mol/L sulfuric acid was added to stop the reaction. After the reaction was stopped, the absorbance at 450 nm was immediately measured by a plate reader.
The results are shown in Fig. 1. According to this test, the WGA lectin, which did not react with the solid-phased antibody and was not affected by NHS added to the sample, was selected as the lectin for detection.

Using the above-mentioned Lectin-ELISA method, serum samples of actual HBV-infected patients before and after treatment were measured.

### < Patient attributes >

### CHB (chronic patient serum):

Sera from untreated (no treatment history with nucleic acid analogs) chronic hepatitis B patients (HBs antigen positivity persisting for 6 months or more) (n=4) were used before (0 weeks) and 48 weeks after nucleic acid analog treatment.
1) HBsAg antibody (Hyb-824, 5 µg/mL) diluted with BSA (5 µg/mL)/PBS was added to a Nunc Immobilizer Amino Plate in an amount of 0.1 mL per well, and the plate was shaken at room temperature for 2 hours to immobilize the antibody. After washing twice with PBS-T, 0.3 mL of TBS was added per well and the wells were allowed to stand at 4°C overnight for blocking.
2) After washing the wells with PBS-T, patient samples (before treatment and 48 weeks after treatment) were diluted 1/20 and 1/100 with Sample dilution buffer (3% BSA/TBS-T), and 0.1 mL was added per well, and the plate was shaken at room temperature for 1.5 hours to capture the antigen onto the antibody-immobilized plate. (As controls, recombinant M-HBs antigen and purified HBs antigen were also diluted in Sample dilution buffer (3% BSA/TBS-T) containing 1% NGS or 5% NGS and captured on the plate.)
3) After washing the wells four times with PBS-T, the wells were reacted with WGA-biotin (VL) diluted to 1 µg/mL with PBS-T at room temperature for 1.5 hours by shaking.
4) After washing the wells four times with PBS-T, the wells were reacted with Peroxidase-conjugated Streptavidin (1/20,000 dilution) diluted with PBS-T at room temperature for 1 hour by shaking.
5) After washing the wells four times with PBS-T, 0.1 mL of 1Step Ultra TMB-ELISA was added per well and allowed to react for 6 minutes, then 50 µL of 1 mol/L sulfuric acid was added to stop the reaction. After the reaction was stopped, the absorbance at 450 nm was immediately measured by a plate reader.

The results are shown in Fig. 2. In the samples before the treatment, by the glycosylated HBs antigen was detected with good sensitivity by the novel Lectin-ELISA method. In the samples after the treatment, it was possible to measure the changes thereof sensitively. According to this, it was found out that the Lectin-ELISA method in the present invention is a method that can detect the status of HBV particles (added sugar chains) in the patient's serum, and can measure the changes due to the effect of treatment.

In the same patient attributes as mentioned above, for untreated (no treatment history with nucleic acid analogs) chronic hepatitis B (HBs antigen positivity persisting for 6 months or more) patients (n=20) as a subject, glycosylated S-HBs was measured using sera before (0 weeks) and 48 weeks after nucleic acid analog treatment, and compared with existing HBsAg (S-HBs). The results are shown in Fig. 3. When the correlation between the ratio of glycosylated S-HBs to S-HBs (Glyco-S/S) at the baseline before the treatment and the changed value in HBsAg (LogU/mL) before and after the treatment was examined by Pearson's analysis, then a significant correlation was admitted that the lower Glyco-S/S ratio before the treatment, the lower the lowering degree in HBsAg (p=0.0158, r=0.5317). That is, it can be considered that the smaller the ratio of glycosylation of S-HBs protein, the better the response to the treatment.

From the above-mentioned results, according to the present invention, it can be understood that the glycosylated S-HBs in blood can be quantitatively detected. Further, by examining the ratio of the glycosylated S-HBs/S-HBs, an index for prophylaxis, treatment or diagnosis of HBV can be obtained.

### UTILIZABILITY IN INDUSTRY

According to the method for measuring the glycosylated S-HBs of the present invention, it was found that a glycosylated S-HBs in blood can be measured by an immunological method. From these results, it can be admitted that the method for measuring the glycosylated S-HBs is useful for diagnosis which contributes to the treatment of HBV infection.

## Claims

1. A method which is a method for detecting hepatitis B virus (HBV) comprising a step of bringing a sample into contact with an antibody which binds to a surface antigen protein, and a lectin.

2. The detection method according to Claim 1, wherein the surface antigen protein of hepatitis B virus is an S protein.

3. The detection method according to Claim 1 or 2, wherein the lectin is wheat germ agglutinin.

4. The detection method according to any one of Claims 1 to 3, which includes a step of measurement by an ELISA method.

5. The detection method according to any one of Claims 1 to 4, which is for testing, prophylaxis or planning treatment of hepatitis B virus.

6. The detection method according to any one of Claims 1 to 5, for screening medicines for testing, prophylaxis or treatment of hepatitis B virus.

7. The detection method according to any one of Claims 1 to 6, which further includes a step of calculating a glycosylated surface antigen protein/surface antigen protein.

8. The detection method according to any one of Claims 1 to 7, wherein the sample is derived from a patient who is receiving or has received nucleic acid analog therapy.

9. The detection method according to any one of Claims 1 to 8, wherein the sample is derived from a patient whose HBV-DNA suppression in blood has been confirmed.

10. A kit for detecting a glycosylated surface antigen protein of hepatitis B virus which comprises an antibody that binds to the glycosylated surface antigen protein of hepatitis B virus, and a lectin.

11. A method for treating or preventing hepatitis B, which includes the method described in any one of Claims 1 to 9.
